# NEW EUROPEAN PATENT SPECIFICATION

(11) **EP 2 144 860 B2**
(45) Date of publication and mention of the opposition decision: **24.12.2014**
(45) Mention of the grant of the patent: 24.08.2011
(21) Application number: 08749983.6
(22) Date of filing: 02.05.2008
(51) Int. Cl.: C07C 1/24, C07C 11/04

(54) **DEHYDRATION OF ALCOHOLS OVER TUNGSTOSILICIC ACID SUPPORTED ON SILICA**
AUF KIESELSÄURE ERFOLGENDE DEHYDRIERUNG VON ALKOHOLEN ÜBER KIESELWOLFRAMLSÄURE
DESHYDRATATION D'ALCOOLS SUR UN ACIDE TUNGSTOSILICIQUE PORTE SUR DE LA SILICE

(30) Priority: 11.05.2007 EP 07251955
(43) Date of publication of application: 20.01.2010
(73) Proprietor: Ineos Sales (UK) Limited, Lyndhurst Hampshire SO43 7FG (GB)
(72) Inventor: POTTER, Nigel, Keith, Dunblane FK15 9HU (GB); SMALL, Karen, West Lothian EH49 6EJ (GB); TABATABAEI, Javad, Yarm TS15 9HQ (GB)
(74) Representative: King, Alex
(86) International application number: PCT/EP2008/055414
(87) International publication number: WO 2008/138775

(56) References cited:
- WO-A-2007/003899
- WO-A-2007/063279
- WO-A1-2007/003910
- WO-A1-2007/063279
- WO-A1-2007/063280
- WO-A1-2007/063281
- WO-A1-2008/062157
- WO-A2-2007/063282
- US-A- 3 974 232
- US-B2- 6 794 535
- K. OTHMER: 'Encyclopedia of Chemical Technology', vol. 9, 1984, JOHN WILEY & SONS, NEW YORK pages 369 - 370

## Description

The present invention relates to a process for the dehydration of alcohols to the corresponding olefins, and in particular, using heteropolyacid catalysts.

The dehydration of alcohols to olefins catalysed using a wide variety of acid catalysts is well-known.

WO 2007/003899 and WO 2007/003901, for example, describe reactive distillation processes for the dehydration of mixed alcohols. The preferred catalysts are heteropolyacids, most preferably impregnated on a support at a loading of 30 to 50 wt% based on the total weight of heteropolyacid and support. For a heteropolyacid support with a surface area of 315 m²/g (typical for Grace 57 silica which is one of the preferred supports in WO 2007/03899 and WO 2007/003901), this range corresponds to a loading of approximately 1.4 to 3.2 mg/m² of catalyst support surface area.

It has now been found that significant advantages are found in alcohol dehydration by supported heteropolyacid catalysts by selection of specific catalyst loadings. In particular, it has surprisingly been found that over a relatively narrow range of loadings improved ethylene productivity and reduced carbon formation on the catalyst is obtained.

Accordingly, in a first aspect the present invention provides a process for the dehydration of one or more alcohols, which process comprises contacting one or more alcohols in the presence of one or more ethers with a supported heteropolyacid catalyst, characterised in that:
(i) the heteropolyacid is tungstosilicic acid in the form of the free acid and supported on a silica support,
(ii) said supported heteropolyacid catalyst has a heteropolyacid loading of from 0.5 to 0.9 mg/m² of catalyst support surface area, and
(iii) that said process comprises passing the one or more alcohols and one or more ethers in the vapour phase over a bed of the supported heteropolyacid catalyst at a temperature between 200°C and 280°C, wherein the feed to the process of the present invention comprises less than 60wt% ethers and at least 30wt% alcohols.

Preferably, the one or more alcohols is a mixture of alcohols, especially comprising ethanol and propanol. Higher alcohols may also be present, but preferably the heaviest alcohols are C6 alcohols.

The one or more alcohols may be provided as any suitable feed comprising said alcohols. The feed may also comprise one or more ethers, such as diethyl ether, dipropyl ether and ethyl propyl ether.

The feed may also comprise water, typically in an amount of less than 10wt%, for example 1-7wt%.

Heteropolyacids usually have a high molecular weight e.g. in the range from 700-8500 and include dimeric complexes.

The heteropolyacid in the present invention is in the form of the free acid (or at least predominantly in the form of the free acid, by which is meant that at least 50% of the heteropolyacid present is in the form of the free acid).

The heteropolyacid used in the process of the present invention is tungstosilicic acid in the form of the free acid and supported on a silica support. For avoidance of doubt, this includes its compositional analogues.

The heteropolyacid is used on a support at an acid loading of 0.5 to 0.9 mg/m² of catalyst support surface area.

Preferably the acid loading is at least 0.6 mg/m² such as at least 0.7 mg/m² of catalyst support surface area.

Preferably the acid loading is less than 0.85 mg/m², such as less than 0.8mg/m², of catalyst support surface area.

Catalyst support surface area, as used herein, refers to BET surface area, as measured according to ASTM D 3663-03, with measurement of 50 points on the adsorption and desorption isotherms, and a degassing temperature of 250°C.

As noted above, it has now been found that significant advantages are obtained in alcohol dehydration by supported heteropolyacid catalysts by selection of the specific catalyst loadings. In particular, it has surprisingly been found that over a relatively narrow range of loadings improved ethylene productivity and reduced carbon formation on the catalyst is obtained.

Preferred silica supports may be porous synthetic silicas in the form of extrudates or pellets, for example as described in EP 0 704 240 A1.

The support is suitably in the form of particles having a diameter of 2 to 10 mm, preferably 4 to 6 mm. The support suitably has a pore volume in the range from 0.3-1.2 ml/g, preferably from 0.6-1.0 ml/g. The support suitably has a crush strength of at least 2 Kg force, suitably at least 5 Kg force, preferably at least 6 Kg and more preferably at least 7 Kg. The crush strengths quoted are based on average of that determined for each set of 50 particles on a CHATTILLON tester which measures the minimum force necessary to crush a particle between parallel plates.

The support suitably has an average pore radius (prior to use) of 10 to 500 Angstroms, preferably an average pore radius of 40 to 180 Angstroms.

An example of a particularly preferred support is Grace 57 silica.

Grace 57 silica has a bulk density of approximately 0.4 g/l and a surface area of approximately 315 m²/g, and thus the range 0.5 to 0.9 mg/m² corresponds to an approximate loading of 13 to 22 wt% of the heteropolyacid based on the total weight of heteropolyacid and support.

In one embodiment, therefore, the supported heteropolyacid catalyst used for the present invention preferably has a heteropolyacid loading comprising 13-22 wt% of the heteropolyacid based on the total weight of heteropolyacid and support, and more preferably comprises 15 to 21 wt% of heteropolyacid based on the total weight of heteropolyacid and support.

In order to achieve optimum performance, the support is suitably free of extraneous metals or elements which might adversely affect the catalytic activity of the system.

The synthesis of heteropolyacids, and their impregnation onto supports where required, may be performed by any known methods, such as those described in EP 0 704 240 A1, EP 1 140 703 B1, WO 2007/003901 and North, E. O.; "Organic Synthesis" 1 p. 129 (1978), published by Huntington N.Y.

If required the heteropolyacid may be purified, for example by using liquid/liquid extraction with a suitable solvent, such as described in EP 1 140 703 B1.

The reaction of the process of the present invention may be performed under any suitable conditions.

Relatively high temperatures are used in the process of the present invention. In particular, the temperature is above 200 °C and less than 280°C.

In a particular embodiment, the temperature is at least 230°C, for example 230°C to 280°C.

It has been found that a relatively low level of alkane formation compared to the desired alkene is obtained when reacting the catalyst of the present invention at relatively high temperatures, even in the presence of ethers in the feed.

As a general rule, ethers in the feed can promote formation of alkanes. Thus, reacting a feed comprising ethanol without diethyl ether being present results in relatively low formation of ethane in ethylene whereas reaction of a feed which is essentially diethyl ether and water in the absence of ethanol will still produce ethylene but will result in the formation of large quantities of ethane in the produced ethylene. Contrary to what is suggested from these results it has been found that significant quantities of ethers can be tolerated in the feed comprising one or more alcohols according to the present invention without significantly increased alkane make compared to absence of the ethers.

This tolerance to ethers and reduced alkane make gives significant process advantages. Firstly, this results in a product stream which requires less purification of the desired olefins in the first place, simplifying downstream processing. Secondly, the process of the present invention usually produces a product stream comprising the desired olefins (dehydration product), unreacted alcohols and ethers, and the unreacted alcohols and ethers can be recycled to the process without significantly affecting the product quality.

In addition, the use of relatively high temperatures can provide improved selectivity to alkene compared to ethers in the first place, minimizing the quantity of ethers formed and subsequently required to be recycled.

The reaction may be performed at atmospheric or elevated pressure. Preferably, the reaction is performed at elevated pressure, especially at least 5 barg, more preferably at least 10 barg. Preferably, the pressure is less than 40 barg.

The reaction is performed at any suitable gas hourly space velocity. Preferably, the reaction is performed at a liquid hourly space velocity (LHSV) in the range 1 to 10 (ml feed/ml catalyst/hour).

### Examples

### Catalyst Preparation

12-tungstosilicic acid.xH2O (H4SiW12O40.xH2O, x being about 24) was used as Acid A.

For catalyst preparation using Acid A, samples of Acid A were dissolved in distilled water to which was added orthophosphoric acid. Grace 57 silica (specific surface area of 310.5 m²/g) was then added in the amounts required for the desired acid loadings. Each sample was allowed to soak for 24 hours, and after soaking the catalyst was drained of excess solution for 1 hour and then dried in air for 16 hours at 105°C.

For K modification (for comparative examples), samples of Acid A were dissolved in distilled water to which was added orthophosphoric acid. In a separate container KHCO3 was dissolved in water and then slowly added, with stirring, to the acid solution. (The quantity of KHCO3 was chosen so as to provide one molar equivalent of potassium per mole of 12-tungstosilicic acidxH2O dissolved in the solution). The above solution was stirred for 15 minutes after the evolution of CO2 had ceased. This is used as Acid B.

For catalyst preparation using Acid B, samples of the above solution of Acid B were added to Grace 57 silica in the amounts required for the desired acid loadings. Each sample was allowed to soak for 24 hours, and after soaking the catalyst was drained of excess solution for 1 hour and then dried in air for 16 hours at 105°C.

Catalysts were prepared as follows:

| Catalyst Number | Acid | Acid loading (mg/m²) |
|---|---|---|
| 1 | A | 0.50 |
| 2 | A | 0.62 |
| 3 | A | 0.77 |
| 4 | A | 0.92 |
| 5 | B | 0.51 |
| 6 | B | 0.61 |
| 7 | B | 0.77 |
| 8 | B | 0.90 |
| 9 | B | 1.09 |

### Catalyst Testing

### Atmospheric pressure tests

Catalysts were screened at atmospheric pressure.

A feed comprising 10% ethanol in helium was passed in the vapour phase over the catalyst for a period of 12 hours at a temperature of 210°C, and then replaced with a feed comprising 5% diethyl ether (DEE) in helium for a period of 5 days, again at 210°C. The feed was then returned to 10% ethanol in helium for 12 hours at 210°C, followed by a final 12 hours at 190°C. A liquid hourly space velocity of 1.3 ml/ml/hr was used throughout.

### Elevated pressure tests

Several catalysts were also tested at elevated pressure.

The test equipment for elevated pressure tests consisted of a Hastelloy fixed bed reactor, which was electrically heated via two independently controlled heating zones. A charge of 50ml catalyst was loaded into the reactor such that it was entirely contained within the heated zones. Liquids were fed to the reactor via a constametric HPLC pump via a trace heated line, entering the reactor as a vapour. Liquid products were collected in a catch-pot and gaseous products passed to a wet gas meter to measure the volume of gas generated. Analysis of liquid and gaseous samples was via Gas Liquid Chromatography for organic components, and Karl Fischer titration for water content.

A feed comprising 58wt% ethanol, 38wt% diethyl ether (DEE) and 4wt% water (which represents a feed for a process with recycle of ethers and unreacted alcohol) was passed in the vapour phase to the reactor at a pressure of 20 barg and liquid hourly space velocities of 1-9 ml/ml/hr. Experiments were performed at reactor inlet temperature of 200-260°C over a period of at least 300 hours.

### Results

### Atmospheric pressure tests

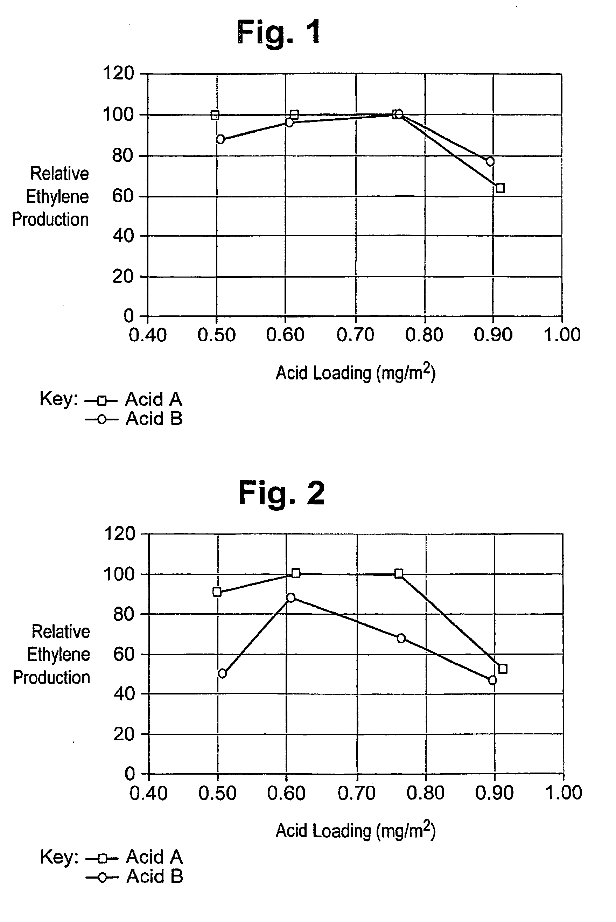

Results for ethylene production from the feed comprising 10% ethanol in helium are shown in Figure 1 after the first 12 hours and in Figure 2 after 5 days. (Both graphs and both catalysts are normalised to 100 based on the maximum ethylene production from catalysts with Acid A in Figure 1.) It can be seen that an optimum in ethylene productivity is observed within the acid loading range of the present invention, the optimum being particularly pronounced after the longer time on stream shown in Figure 2, showing both an improvement in ethylene productivity and a reduced deactivation rate within the range of the present invention.

The graphs also show a surprising improvement in catalyst stability when using a supported tungstosilicic acid with no cation modification (Acid A). In particular, catalysts of Acid A do not deactivate to any measurable extent for HPA loadings of 0.62 and 0.77 mg/m²

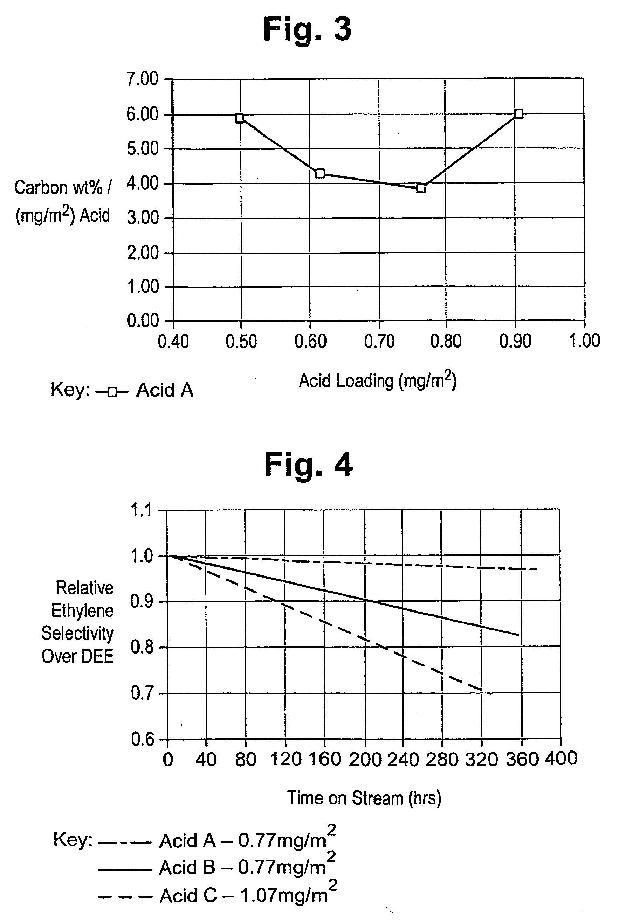

Figure 3 represents carbon analysis results from Catalyst A after removal from the reactor. Reduced carbon lay-down is observed within the range of acid loading of the present invention, (with a minimum between 0.5 to 0.9 mg/m² of catalyst support surface area) consistent with the reduced deactivation rate shown by comparison between Figures 1 and 2.

### Elevated pressure tests

Results for ethylene selectivity versus time on stream are shown in Figure 4 for three catalysts. The results are normalised to highlight the differences in the relative rate of deactivation. The results are consistent with those shown in Figures 1 to 3 at atmospheric pressure. In particular, it can be seen that the catalyst of Acid A (catalyst 3) with a loading of 0.77 mg/m² shows lower deactivation than the two catalysts of Acid B, whether at the same loading of 0.77 mg/m² (catalyst 7) or with a higher loading.

The catalyst according to the present invention also produced a product with relatively low ethane impurity in the produced ethylene. In particular, the ethane in ethylene was below 500ppm throughout the 360 hours af the experiment.

### Alkane make versus Temperature

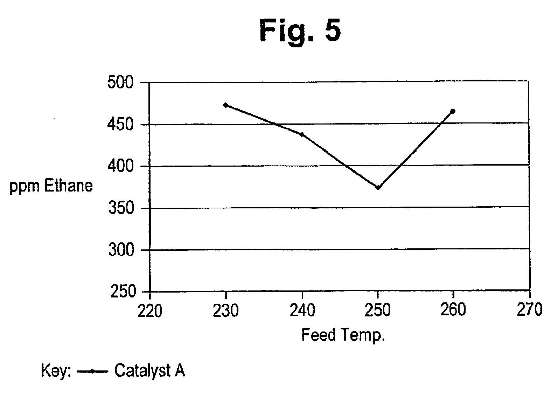

Figure 5 shows the ethane production from Catalyst A as tested at a liquid hourly space velocity of 6 ml/ml/hour, with recycle of unreacted alcohols and ethers at elevated pressure of 2 MPa. The feed composition was 58wt% ethanol, 38wt% diethyl ether (DEE) and 4wt% water (which represents a potential feed for a process with recycle of ethers and unreacted alcohol).
Figure 5 shows that despite the elevated temperatures, across the range tested the ethane production is maintained below 500 ppm, whereas an increase in ethane make with temperature might have been expected.
Figure 5 also shows that the amount of ethane produced was relatively low despite the fact that ethers are present in the feed, whereas increased ethane production might again have been expected from the presence of ethers in the feed. This is further illustrated in the following example.

### Effect of diethyl ether co-feed

Experiments were performed as for the elevated pressure tests detailed above, with a space velocity of 6 ml/ml/hour and at a pressure of 2 MPa, except that three experiments were performed with different feeds over the same catalyst at 240°C to investigate the effect of the feed composition on the ethane impurity in the ethylene produced.

Feed 1 was a feed comprising essentially diethylether, comprising 97wt% dimethyl ether and 3wt% water.

Feed 2 had no diethylether and comprised 95.4wt% ethanol and 4.6wt% water.

Feed 3 comprised 58wt% ethanol, 38wt% diethyl ether (DEE) and 4wt% water (which represents a potential feed for a process with recycle of ethers and unreacted alcohol).

Feed 1 resulted in the formation of 1169 ppm ethane in ethylene and feed 2 resulted in the formation of 466 ppm ethane in ethylene. Comparison of these results shows that reaction of diethyl ether results in significantly increased formation of ethane in the product ethylene. Feed 3, however, resulted in only 463 ppm of ethane in ethylene despite the presence of significant quantities of diethyl ether in the feed. Contrary to what is suggested from the result using diethyl ether and water only (Feed 1), therefore, these examples show that nearly 40wt% of diethyl ether can be tolerated without significantly increased alkane make compared to absence of diethyl ether.

## Claims

1. A process for the dehydration of one or more alcohols, which process comprises contacting one or more alcohols in the presence of one or more ethers with a supported heteropolyacid catalyst, **characterised in that**:
(i) the heteropolyacid is tungstosilicic acid in the form of the free acid and supported on a silica support,
(ii) said supported heteropolyacid catalyst has a heteropolyacid loading of from 0.5 to 0.9 mg/m² of catalyst support surface area, and
(iii) that said process comprises passing the one or more alcohols and one or more ethers in the vapour phase over a bed of the supported heteropolyacid catalyst at a temperature between 200°C and 280°C, wherein the feed to the process of the present invention comprises less than 60wt% ethers and at least 30wt% alcohols.

2. A process as claimed in claim 1 wherein said supported heteropolyacid catalyst has a heteropolyacid loading of from 0.6 to 0.85 mg/m² of catalyst support surface area.

3. A process as claimed in claim 1 or claim 2 wherein the one or more alcohols comprises ethanol, and optionally propanol.

4. A process as claimed in any one of the preceding claims wherein the temperature in step (iii) is at least 230°C.

5. A process as claimed in any one of the preceding claims wherein step (iii) produces a product stream comprising olefins, unreacted alcohols and ethers, and the unreacted alcohols and ethers are recycled to the process.

6. A process as claimed in any one of the preceding claims wherein step (iii) is performed at an elevated pressure of 5 to 40 barg.

7. A process as claimed in any one of the preceding claims wherein the heteropolyacid is used on a support at an acid loading of 13 to 22 wt% of the heteropolyacid based on the total weight of heteropolyacid and support.

8. A process for the dehydration of one or more alcohols, which process comprises contacting one or more alcohols comprising ethanol, and optionally propanol, in the presence of one or more ethers with a supported heteropolyacid catalyst, **characterised in that**:
(i) the heteropolyacid is tungstosilicic acid in the form of the free acid and supported on a silica support,
(ii) said supported heteropolyacid catalyst has a heteropolyacid loading of from 0.6 to 0.85 mg/m² of catalyst support surface area,
(iii) that said process comprises passing the one or more alcohols and one or more ethers in the vapour phase over a bed of the supported heteropolyacid catalyst at a temperature in the range 230°C to 280°C to produce a product stream comprising olefins (dehydration product), unreacted alcohols and ethers, and
(iv) the unreacted alcohols and ethers in the product stream of step (iv) are recycled to the process.

## Patentansprüche

1. Verfahren zur Dehydratisierung von einem oder mehreren Alkoholen, wobei das Verfahren das In-Kontakt-Bringen von einem oder mehreren Alkoholen in Gegenwart von einem oder mehreren Ethern mit einem geträgerten Heteropolysäure-Katalysator umfasst, **dadurch gekennzeichnet, dass**:
(i) es sich bei der Heteropolysäure um Kieselwolframsäure in Form der freien Säure und geträgert auf einem Siliciumoxidträger handelt;
(ii) der geträgerte Heteropolysäure-Katalysator eine Heteropolysäure-Beladung von 0,5 bis 0,9 mg/m² Katalysatorträgeroberfläche aufweist; und
(iii) das Verfahren das Leiten des einen oder der mehreren Alkohole und des einen oder der mehreren Ether in der Dampfphase über ein Bett des geträgerten Heteropolysäure-Katalysators bei einer Temperatur zwischen 200 °C und 280 °C umfasst, wobei der Zustrom zum Verfahren der vorliegenden Erfindung weniger als 60 Gew.-% Ether und wenigstens 30 Gew.-% Alkohole umfasst.

2. Verfahren gemäß Anspruch 1, wobei der geträgerte Heteropolysäure-Katalysator eine Heteropolysäure-Beladung von 0,6 bis 0,85 mg/m² Katalysatorträgeroberfläche aufweist.

3. Verfahren gemäß Anspruch 1 oder 2, wobei der eine oder die mehreren Alkohole Ethanol und gegebenenfalls Propanol umfassen.

4. Verfahren gemäß einem der vorstehenden Ansprüche, wobei die Temperatur in Schritt (iii) wenigstens 230 °C beträgt.

5. Verfahren gemäß einem der vorstehenden Ansprüche, wobei Schritt (iii) einen Produktstrom erzeugt, der Olefine, nicht umgesetzte Alkohole und Ether umfasst, und die nicht umgesetzten Alkohole und Ether im Kreislauf in das Verfahren zurückgeführt werden.

6. Verfahren gemäß einem der vorstehenden Ansprüche, wobei Schritt (iii) unter einem erhöhten Manometerdruck von 5 bis 40 bar durchgeführt wird.

7. Verfahren gemäß einem der vorstehenden Ansprüche, wobei die Heteropolysäure auf einem Träger mit einer Säurebeladung von 13 bis 22 Gew.-% der Heteropolysäure, bezogen auf das Gesamtgewicht von Heteropolysäure und Träger, verwendet wird.

8. Verfahren zur Dehydratisierung von einem oder mehreren Alkoholen, wobei das Verfahren das In-Kontakt-Bringen von einem oder mehreren Alkoholen, die Ethanol und gegebenenfalls Propanol umfassen, in Gegenwart von einem oder mehreren Ethern mit einem geträgerten Heteropolysäure-Katalysator umfasst, **dadurch gekennzeichnet, dass**:
(i) es sich bei der Heteropolysäure um Kieselwolframsäure in Form der freien Säure und geträgert auf einem Siliciumoxidträger handelt;
(ii) der geträgerte Heteropolysäure-Katalysator eine Heteropolysäure-Beladung von 0,6 bis 0,85 mg/m² Katalysatorträgeroberfläche aufweist;
(iii) das Verfahren das Leiten des einen oder der mehreren Alkohole und des einen oder der mehreren Ether in der Dampfphase über ein Bett des geträgerten Heteropolysäure-Katalysators bei einer Temperatur im Bereich von 230 °C bis 280 °C unter Bildung eines Produktstroms, der Olefine (Dehydratisierungsprodukt), nicht umgesetzte Alkohole und Ether umfasst, umfasst; und
(iv) die nicht umgesetzten Alkohole und Ether im Produktstrom von Schritt (iv) im Kreislauf in das Verfahren zurückgeführt werden.

## Revendications

1. Procédé pour la déshydratation d'un ou plusieurs alcools, lequel procédé comprend la mise en contact d'un ou plusieurs alcools en présence d'un ou plusieurs éthers avec un catalyseur à hétéropolyacide supporté, **caractérisé en ce que** :
(i) l'hétéropolyacide est l'acide tungstosilicique sous forme d'acide libre et supporté sur un support de silice,
(ii) ledit catalyseur à hétéropolyacide supporté a un chargement d'hétéropolyacide de 0,5 à 0,9 mg/m² d'aire de surface de support de catalyseur, et
(iii) ledit procédé comprend le passage du un ou plusieurs alcools et du un ou plusieurs éthers dans la phase vapeur au-dessus d'un lit du catalyseur à hétéropolyacide supporté à une température entre 200°C et 280°C, où l'alimentation du procédé de la présente invention comprend moins de 60 % en poids d'éthers et au moins 30 % en poids d'alcools.

2. Procédé selon la revendication 1 où ledit catalyseur à hétéropolyacide supporté a un chargement d'hétéropolyacide de 0,6 à 0,85 mg/m² d'aire de surface de support de catalyseur.

3. Procédé selon la revendication 1 ou la revendication 2 où le un ou plusieurs alcools comprend l'éthanol, et éventuellement le propanol.

4. Procédé selon l'une quelconque des revendications précédentes où la température dans l'étape (iii) est au moins 230°C.

5. Procédé selon l'une quelconque des revendications précédentes où l'étape (iii) produit un courant de produit comprenant des oléfines, des alcools et des éthers qui n'ont pas réagi, et les alcools et les éthers qui n'ont pas réagi sont recyclés dans le procédé.

6. Procédé selon l'une quelconque des revendications précédentes où l'étape (iii) est accomplie à une pression élevée de 5 à 40 barg.

7. Procédé selon l'une quelconque des revendications précédentes où l'hétéropolyacide est utilisé sur un support à un chargement d'acide de 13 à 22 % en poids de l'hétéropolyacide sur la base du poids total d'hétéropolyacide et de support.

8. Procédé pour la déshydratation d'un ou plusieurs alcools, lequel procédé comprend la mise en contact d'un ou plusieurs alcools comprenant de l'éthanol, et éventuellement du propanol, en présence d'un ou plusieurs éthers avec un catalyseur à hétéropolyacide supporté, **caractérisé en ce que** :
(i) l'hétéropolyacide est l'acide tungstosilicique sous forme d'acide libre et supporté sur un support de silice,
(ii) ledit catalyseur à hétéropolyacide supporté a un chargement d'hétéropolyacide de 0,6 à 0,85 mg/m² d'aire de surface de support de catalyseur,
(iii) ledit procédé comprend le passage du un ou plusieurs alcools et du un ou plusieurs éthers dans la phase vapeur au-dessus d'un lit du catalyseur à hétéropolyacide supporté à une température dans la plage de 230°C à 280°C pour produire un courant de produit comprenant des oléfines (produit de déshydratation), des alcools et des éthers qui n'ont pas réagi, et
(iv) les alcools et les éthers qui n'ont pas réagi dans le courant de produit de l'étape (iv) sont recyclés dans le procédé.
